(19)

Europäisches
Patentamt
European
Patent Office
Office européen
des brevets

(11) **EP 4 202 943 A1**

(12) **EUROPEAN PATENT APPLICATION**

(43) Date of publication:
**28.06.2023 Bulletin 2023/26**

(21) Application number: **21217609.3**

(22) Date of filing: **23.12.2021**

(51) International Patent Classification (IPC):
**G16H 10/60** $^{(2018.01)}$

(52) Cooperative Patent Classification (CPC):
**G16H 10/60**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Sensyne Health Group Limited
Oxford OX4 4GE (GB)**

(72) Inventors:
• **JAVER, Avelino
  Oxford, OX4 4GE (GB)**

• **ALBERGANTE, Luca
  Oxford, OX4 4GE (GB)**
• **BAXTER, Janie
  Oxford, OX4 4GE (GB)**
• **AZQUETA-GAVALDON, Andres
  Oxford, OX4 4GE (GB)**
• **WALLIS, Jamie
  Oxford, OX4 4GE (GB)**
• **DÜRICHEN, Robert
  Oxford, OX4 4GE (GB)**

(74) Representative: **Korenberg, Alexander Tal et al
Kilburn & Strode LLP
Lacon London
84 Theobalds Road
London WC1X 8NL (GB)**

(54) **METHOD AND SYSTEM FOR FINDING MISSING VALUE FOR PHYSIOLOGICAL FEATURE**

(57)     A method for determining a missing value of a physiological feature of interest for a target patient is disclosed. The method comprises accessing, for a plurality of recorded patients, patient information including recorded values for a plurality of features including the physiological feature of interest and one or more additional features. Target patient information for the target patient is obtained, comprising values for the one or more additional features. For each recorded patient, the method comprises determining a measure of similarity between said patient and the target patient based on the one or more additional features. A subset of patients which are similar to the target patient are selected, based on the measures of similarity. If the difference between a population measure for the subset and for the plurality of recorded patients is statistically significant, the method comprises assigning a value to the feature of interest for the target patient based on the values of the physiological feature of interest of the subset of patients.

FIG. 1

EP 4 202 943 A1

## Description

## Technical Field

This disclosure relates generally to filling in a missing value, and specifically to a computer-implemented method and system for finding a missing value for a physiological feature of interest. The missing value may be used for medical diagnosis and/or identifying a treatment regime, for example.

## Background

In recent years, there have been significant efforts in constructing Medical Real World Evidence (RWE) by combining information stored in Electronic Patient Records (EPRs). Medical RWE has a significant role in biomedical research, and can be used to identify potential physio-pathological pathways associated with a range of diseases which can be used to propose novel drug targets. Moreover, medical RWE can be used to assist in the diagnosis of diseases, as new connections or correlations between physiological features can be recognized. In particular, medical RWE may be used to better characterize heart failure (HF) patients and to explore long-term effects of different medications and treatments across patients with a range of comorbidities.

However, EPRs can be incomplete, and may lack particular features of interest. Features may be missing from a patient's EPR, for example due to a failure to record information, or due to technical or administrative reasons. The collection of such data also has inherent limitations, resulting in a lack of information about key features of patients, particularly when these features cannot be encoded using commonly used standards such as ICD-10.

Incomplete EPRs can reduce the usefulness of medical RWE, as important information may not be taken into consideration. There is therefore a need to improve the completeness of this data.

In particular, EPRs may be used to classify heart failure patients based on ejection fraction (EF) (e.g. the volumetric percentage of blood ejected from the left ventricle of the heart during each contraction). Different values of EF are associated with a different severity of disease, ranging from the severe disease of heart failure with reduced EF (HFrEF), to heart failure with mildly reduced EF (HFmrEF), to the less severe disease of heart failure with preserved EF (HFpEF). Research into this field has been limited and primarily focused on descriptive statistics, leading to challenges in identifying patients with a particular EF classification and the usage of an appropriate treatment regimen.

Filling in missing values (also known as imputation) is frequently used to improve data quality and to make data more suitable for a variety of uses, such as the use of certain machine learning models.

Regression is among the most commonly used approach for feature imputation. An appropriate regression model is built using data points (e.g. corresponding to patients) with valid (e.g. complete) measurements for a particular feature, and then used to predict values for the same particular feature for data points (e.g. patients) without a valid measurement for the particular feature.

Multivariate imputation by chained equations (MICE) is another common approach, in which values from multiple imputation models are used to better characterize uncertainty around an imputed value.

However, both of these approaches rely on the use of a model for predicting a target feature from a set of predefined input features, and focus on predicting the target feature for all target patients. Such models, which often may be too simplistic to capture the data or have complex non-linear modalities, require a large amount of data in order to allow for proper fitting. A large amount of data may not always be available. Additionally, models such as these allow construction of a function which maps input features to a specific output, using prespecified interaction modalities between variables. These modalities may be made arbitrarily complex but are often unable to capture complex non-linear interactions. Moreover, such models assume that all of the relevant features expected by the imputation method have been measured. This assumption may cause problems when the data is composed of unknown subgroups with different mechanisms influencing the features of interest, e.g. different groups of patients who develop the same condition but as a result of different causes. In such cases, key input features associated with the feature of interest may be missed. Additionally, as these approaches focus on predicting the target feature for all target patients, target features may be predicted regardless of the likelihood of a correct prediction. In other words, these approaches assign a predicted value to a feature regardless of the quality of the predictions, including assigning poor predictions to a target feature..

It would therefore be advantageous to provide a method for filling in missing values which seeks to address these and other disadvantages encountered in the state of the art.

## Summary

Aspects and optional features of the present disclosure are described in the accompanying claims.

One aspect of the present disclosure provides a computer-implemented method for determining a missing value of a physiological feature of interest for a target patient. The method comprises accessing patient information for a plurality of recorded patients, the patient information comprising, for each patient of the plurality of recorded patients, recorded values for a plurality of features. The plurality of features includes the physiological feature of interest and one or more additional features. The method further comprises obtaining target patient information for the target patient, the target patient information comprising values for the one or more additional features. For each patient of the plurality of recorded patients, a respective measure of similarity between said patient and the target patient based on the one or more additional features is determined and a subset of patients of the plurality of recorded patients that are similar to the target patient based on the respective one or more measures of similarity is selected. The method comprises determining if a difference between a population measure, for example an overrepresented category, of the recorded values corresponding to the physiological feature of interest for the subset of the plurality of recorded patients that are similar to the target patient and the population measure of the recorded values corresponding to the physiological feature of interest for the plurality of recorded patients is statistically significant using a statistical test. The method comprises, if it is determined that the difference is statistically significant, assigning a value to the physiological feature of interest for the target patient based on the recorded values of the physiological feature of interest for the subset of patients of the plurality of recorded patients that are similar to the target patient.

Optionally, the physiological feature of interest may be a categorical feature. For example, the missing value may indicate whether a numeric value of a physiological measurement corresponding to the physiological feature of interest is within a predefined numerical range. Assigning the value to the physiological feature of interest for the target patient may comprise assigning the category of the categorical feature of the majority of the subset of patients of the plurality of recorded patients that are similar to the target patient as the missing value.

Optionally, the physiological feature of interest may be an ejection fraction for heart failure, the ejection fraction being a volumetric fraction of fluid ejected from a chamber of a heart with each contraction. For example, the missing value may indicate if the ejection fraction is a preserved ejection fraction, a reduced ejection fraction or, in some implementations, a mildly reduced ejection fraction.

In some implementations, the patients may be heart-failure patients, for example patients with chronic heart failure, and the method may comprise diagnosing the target patient as one of preserved ejection fraction (HFpEF), for example ejection fraction >50%, and reduced ejection fraction (HFrEF), for example ejection fraction ≤50% or ≤ 30%) and, optionally in some implementations mildly reduced ejection fraction (HFmrEF), for example 30% < ejection fraction ≤ 50%. In some implementations, the method comprises recommending a treatment option based on the categorical value / diagnosis.

Optionally, the one or more measures of similarity may comprise a distance between the respective one or more additional features in a corresponding feature space, for example a Jaccard distance, a Manhattan distance and/or a Gower distance.

In another aspect of the present disclosure, one or more transitory or non-transitory computer-readable media are provided. The computer-readable media comprise instructions which, when executed by a computer, cause the computer to carry out any of the methods disclosed herein. Other aspects provide a computer program product comprising instructions which, when executed by a computer, cause the computer to carry out any of the methods disclosed herein.

In another aspect of the present disclosure, a device is provided which comprises a transmission subsystem configured to transmit data, a receiving subsystem configured to receive data, one or more processors, and a memory comprising instructions which, when executed cause the one or more processors to execute any of the methods disclosed herein. Further aspects provide a system having a memory, one or more computer readable media as described above and a processor configured to implement the instructions.

## Brief description of the drawings

Some specific implementations are now described, by way of example only, with reference to the accompanying drawings, in which:

> Figure 1 illustrates a flowchart of a method of assigning a missing value to a physiological feature of interest for a target patient according to embodiments of the disclosure;
> Figure 2 illustrates a flowchart of a method of diagnosing heart failure with reduced ejection fraction for a target patient according to embodiments of the disclosure;
> Figures 3a-3c illustrate a simplified visualization for assigning a missing value to a physiological feature of interest for a target patient according to embodiments of the disclosure;
> Figure 4 illustrates a block diagram of a computing device; and
> Figure 5 illustrates a diagram of a computer-readable medium.

## Detailed description

Aspects and features of the disclosure will be described below. In overview, and without limitation, the present application relates to a method for finding a missing value of a physiological feature of interest for a target patient using a non-parametric model that accounts for local data, e.g. patient information for a plurality of recorded patients. The method comprises the use of one or more additional features other than the feature of interest in order to identify patients from the plurality of recorded patients which are similar to the target patient. If a population measure of the similar patients is statistically significantly different from that of the population measure for the plurality of recorded patients, the values recorded for the physiological feature of interest for the similar patients may be used to assign a value to the physiological feature of interest for the target patient. The population measure may be an overrepresented category, e.g. a most frequent category or a category with a high occurrence, an occurrence exceeding an expected occurrence.

The methods disclosed herein adopt a probabilistic approach. As the method assigns a value to the physiological feature of interest for the target patient if a difference between the population measure of the subset of similar patients and the plurality of recorded patients is statistically significant, values may only be assigned when the probability of a correct prediction or imputation is sufficiently high. This is in contrast to known methods such as regression or MICE, which predict values for all target patients without consideration of a likelihood of a correct prediction. The probabilistic approach of the methods described herein therefore improves the quality of predicted values by assigning the predicted (e.g. imputed) values only if the likelihood of a correct prediction is sufficiently high (e.g. measured by a statistical significance or similar metric).

Throughout the following description, reference will be made to an example relating to assigning a value of an ejection fraction to a patient with heart failure. It is to be understood that this is merely exemplary - the methods described herein may be applied similarly to a wide range of other physiological features that may be represented in a binary or categorical form.

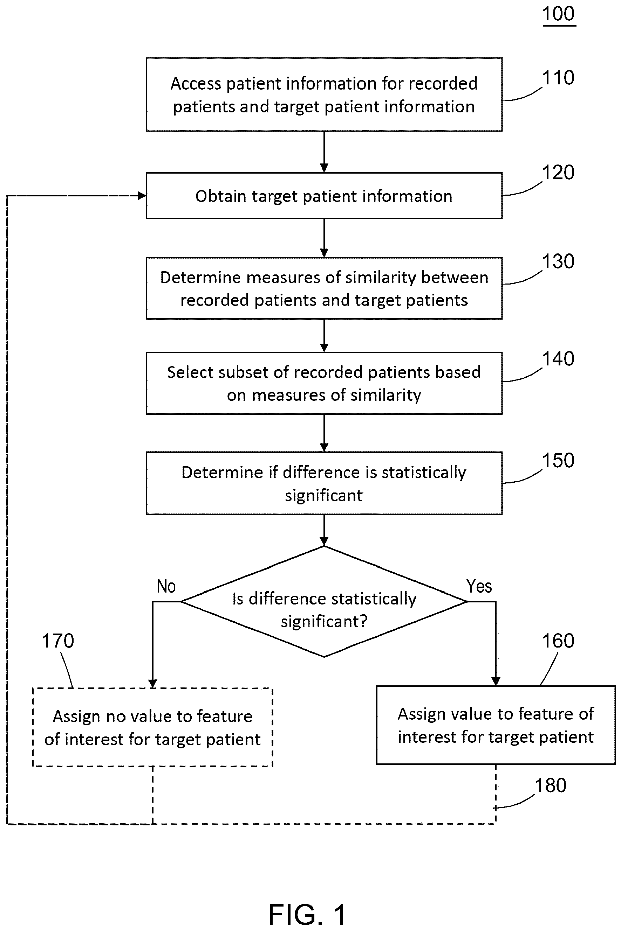

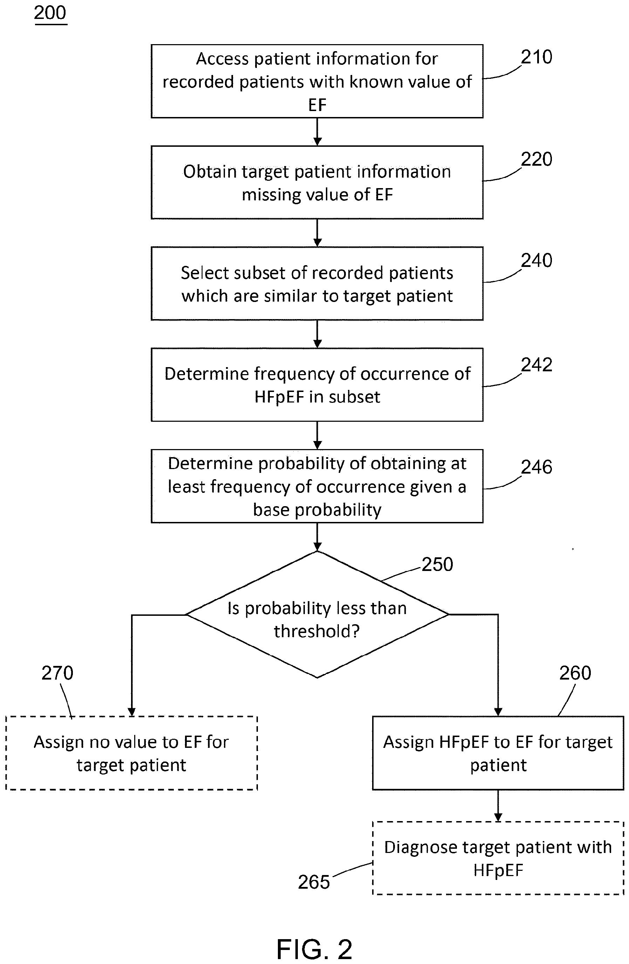

The description of Figure 1 will describe the general method, whilst the description of Figure 2 will describe a specific example in which the general method is applied to the categorisation of ejection fraction in heart failure patients. It is to be understood that elements of the specific example may be applicable in any implementation of the general method. The categorization may be used for diagnosis of types of heart failure as described above and recommendation of treatment option.

**Figure 1** illustrates a flowchart of a method 100 of assigning a missing value to a physiological feature of interest for a target patient.

A target patient is a patient for which a value for a physiological feature of interest is missing, and is to be found, determined, assigned or imputed.

Patient information for a target patient (hereinafter referred to as the target patient information) may be incomplete - that is, the target patient information may be missing a value for a physiological feature of interest. For example, the target patient information may have no value corresponding to an ejection fraction.

The method 100 comprises the operation of accessing 110 patient information for a plurality of recorded patients. Recorded patients may be patients for which a value associated with the physiological feature of interest is known, e.g. recorded. The patient information comprises, for each of the recorded patients, a recorded value for each of a plurality of physiological features, including the physiological feature of interest and one or more additional features. Examples of physiological features may include an ejection fraction (EF), and examples of additional features may include demographic data (e.g. age, sex), vital signs (e.g. beats per minute), laboratory results (e.g. systolic and diastolic blood pressure), and treatment data (e.g. medications, treatments, etc.).

Patient information for the plurality of recorded patients may be accessed via a database, e.g. a remote database or remote server, via local memory, e.g. a database stored locally, from a single source or from a plurality of data sources. The patient information for the plurality of recorded patients may be provided by one or more known datasets, such as one or more research datasets. In some cases, the recorded patients may comprise patients in a particular group, or sharing a particular characteristic or diagnosis. For example, the recorded patients may be heart failure patients having recorded values for the ejection fraction. That is, the plurality of recorded patients may be selected from a large, general database or pool of recorded patients based on the use case.

For each recorded patient of the plurality of recorded patients, the patient data includes a recorded value associated with a physiological feature of interest. In the example in which the physiological feature of interest is an ejection fraction, the patient data would therefore include a value of the ejection fraction for each of the plurality of recorded patients. The particular example relating to an ejection fraction will be discussed in detail with reference to Figure 2.

The method 100 further comprises the operation of obtaining 120 target patient information. The target patient information includes values for one or more additional features, such as those described above, but does not include a value corresponding to the physiological feature of interest. That is, the value of the physiological feature of interest for the target patient is not present (e.g. missing) in the target patient information, e.g. the value of the physiological feature of interest may not have been recorded, and/or may not be known. The target patient information may be obtained from the same data source as the patient information for the plurality of recorded patients, for example when identifying and filling in incomplete entries in a database, or from a different data source. For example, the target patient information may be input directly, such as part of a registration or logging process.

It is noted that operations 110 and 120 may be performed in any order, simultaneously or substantially simultaneously.

The method 100 further comprises the operation of determining 130 measures of similarity between each of the plurality of recorded patients and the target patient. That is, for each recorded patient of the plurality of recorded patients, a measure of similarity is determined based on the target patient values and the recorded patient values for each of the additional features common to the recorded patient and the target patient. For example, if both the target patient and the recorded patient had values for the additional features of age, blood pressure and weight, then a measure of similarity may be based on the similarity of each of these additional features, or a subset of these additional features, individually or in combination. A measure of similarity may be determined by calculating a distance between the values of each additional feature, or a distance in a space having a respective dimension for each additional feature. In some cases, the additional features may be normalized prior to determining the measure of similarity, for example to ensure an equal weighting of the additional features. In this example, the method would comprise determining a measure of similarity based on the difference in age, blood pressure and weight, between the target patient and the values of these features corresponding to the recorded patient.

The measure of similarity may be determined by calculating a distance in a corresponding feature space having a respective dimension for each additional feature. The distance may be determined using, for example, a Euclidean distance, a Jaccard distance, a Manhattan distance and/or a Gower distance.

The method 100 further comprises the operation of selecting 140 a subset of recorded patients which are similar to the target patient based on the respective one or more measures of similarity. That is, the measure of similarity corresponding to each recorded patient (e.g. indicative of a similarity between said recorded patient and the target patient) may be compared to a threshold value, such as a threshold similarity parameter (or hyperparameter). A threshold distance parameter may be used as a threshold similarity parameter. Recorded patients having a measure of similarity greater than or equal to the threshold similarity parameter (e.g. recorded patients having a higher similarity measure than the threshold, for example a shorter distance, and therefore being more similar to the target patient) may be included in the subset of recorded patients.

In cases where the measure of similarity is a distance value, a threshold distance parameter is used. Recorded patients whose distance value (e.g. representing a distance, or dissimilarity, from the target patient) is less than or equal to the threshold distance parameter may be included in the subset of recorded patients, as the distance between such recorded patients and the target patient is smaller (i.e. they are more similar).

The method further comprises the operation of determining 150 if a difference between a subset population measure of the recorded values corresponding to the physiological feature of interest for the subset of the plurality of recorded patients that are similar to the target patient and a population measure of the recorded values corresponding to the physiological feature of interest for the plurality of recorded patients is statistically significant using a statistical test. That is, operation 150 of the method may determine whether a particular value (e.g. categorical value) of the physiological feature of interest is overrepresented (e.g. has a higher occurrence than expectation). Specifically, a statistical test may be applied to determine a probability that the subset of recorded patients and the remaining ones of the plurality of recorded patients are samples drawn from the same distribution over recorded patients with respect to the physiological feature of interest (or in other words, the probability that there is no underlying difference in the respective distributions of values of the physiological feature of interest and that any observed difference, for example as measured by a difference in the population measure, is due to random chance).

A population measure may comprise, for example, a frequency of occurrence of a particular category, or another statistical property used to categorise a population. Statistical tests for various assumed distributions or statistical models can include a binomial test, a chi-square test, a chi-square goodness-of-fit test, and the like.

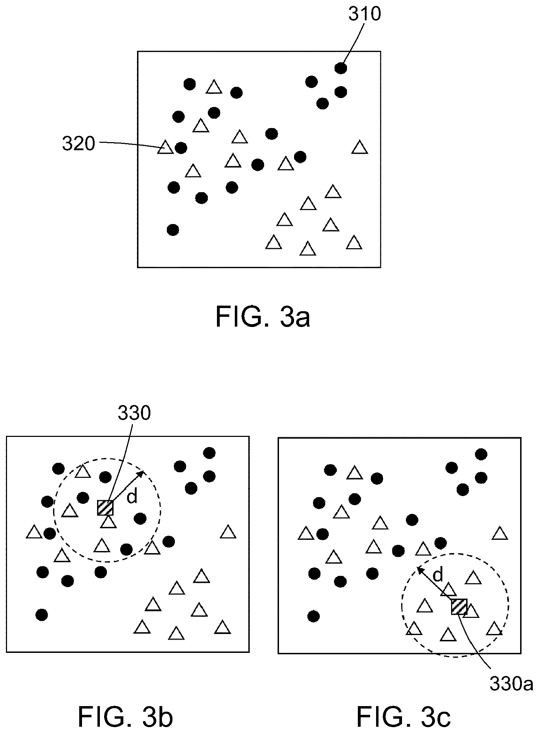

Determining if a difference between the population measure of the subset of patients (e.g. the similar patients) and the corresponding population measure of the plurality of recorded patients (e.g. the more general population of patients) for the physiological feature of interest may comprise comparing the distribution of the recorded values for the physiological feature of interest (e.g. ejection fraction) within the subset of similar patients to the distribution of the recorded values for the physiological feature of interest (e.g. ejection fraction) more generally (e.g. across all of the plurality of recorded patients). Such a comparison would indicate whether the recorded values for the physiological feature of interest in the subset of recorded patients which are similar to the target patient have, for example, a narrower range of values or a higher than expected occurrence of a particular value or classification and are therefore overrepresented. This will be elucidated further with reference to Figures 2 and 3.

In some examples, determining if the difference between the population measures may comprise determining, for the recorded values of the physiological feature of interest:

- a population measure (e.g. frequency of occurrence) for the plurality of recorded patients, and
- the population measure (e.g. frequency of occurrence) for the subset of recorded patients similar to the target patient (as selected in operation 140).

The physiological feature of interest may be a categorical feature, e.g. where the value associated therewith corresponds to one of a plurality of classes or categorizations, which may be mutually exclusive.

Determining whether a difference between the population measures is statistically significant may comprise determining a probability of rejecting the null hypothesis that there is no difference between the subset population of similar recorded patients and the population of the plurality of recorded patients. If the probability of rejecting the null hypothesis is greater than a threshold significance value, the difference may be determined to be statistically significant.

In some cases, the probability of obtaining the distribution observed in the subset of similar patients given the more general distribution associated with the plurality of recorded patients may be compared to a threshold probability value. If the probability of obtaining the distribution that is observed is less than or equal to the threshold probability value, e.g. the distribution that is observed is more unlikely than a threshold value, then the difference between the population measures may be determined to be statistically significant.

The statistical test may comprise, for example, a binomial test (e.g. a one-tailed binomial test), and/or a chi-squared test. The choice of test may depend on the type of value associated with the physiological feature of interest. For example, if the physiological feature of interest is binary in nature (e.g. the associated value is one of two possibilities), a binomial test or a Fisher Exact test may be used. If the physiological feature of interest is categorial (e.g. the associated value is one of a plurality of discrete or class-based possibilities), a chi-squared test or a binomial test (e.g. used in a one-versus-rest approach) may be used. It is to be understood that the tests listed herein are merely exemplary, and any statistical test suitable for determining a statistical significance may be used.

In some cases, a statistical significance, probability or similar metric may be output to a user, e.g. via a display, printout, log, audio signal, image, video or the like.

If the difference between the population measures of the plurality of recorded patients and the subset of recorded patients similar to the target patient is determined to be statistically significant, the method 100 continues to the operation of assigning 160 a value to the physiological feature of interest for the target patient. The value assigned to the physiological feature of interest for the target patient may be based on the recorded values of the physiological feature of interest for the subset of patients of the plurality of recorded patients that are similar to the target patient. For example, the value assigned to the target patient for the physiological feature of interest may be an overrepresented value (e.g. category) among the subset of recorded patients which are similar to the target patient. In some cases, an overrepresented category may correspond to the most frequently occurring category or the category of the majority of the subset, but this is not necessarily the case. That is, the overrepresented category may be a category which has a higher-than-likely occurrence in the subset of recorded patients similar to the target patient in view of a general likelihood of occurrence (e.g. the likelihood of occurrence in the population of recorded patients), even in cases where the overrepresented category is represented by less than 50% of the patients in the subset.

In some examples, the missing value may indicate whether a numeric value of a physiological measurement corresponding to the physiological feature of interest is within a predefined numerical range. That is, the patient information for the plurality of recorded patients may include a numerical value associated with the physiological feature of interest. The numerical value for each of the recorded patients may fall within a numerical range which may be indicative of a categorical value. For example, a value for an ejection fraction for each recorded patient may be a percentage or a fraction. A categorical value of "reduced ejection fraction", for example, may be associated with an ejection fraction of less than or equal to 30%. This will be described further with reference to Figure 2.

In some cases, if the difference between the population measures of the plurality of recorded patients and the subset of recorded patients similar to the target patient is determined to be not statistically significant, then no value is assigned to the physiological feature of interest for the target patient.

By only assigning a value to the physiological feature of interest when the difference between the population measures of the plurality of recorded patients and the subset of recorded patients similar to the target patient is statistically significant, the accuracy of the imputed or assigned value is improved, and unreliable values are not assigned. That is, if there is no statistically significant difference for the values of the physiological feature of interest between the two population groups (e.g. the plurality of recorded patients and the subset of similar patients), then an accurate value is not imputed and inaccurate data is not created. in other words, a value is only assigned for patients when a high likelihood of a correct imputation is determined. In other words, the method 100 provides a probabilistic output which improves the quality of predicted values assigned to the physiological feature of interest.

The method 100 may be used to impute or assign missing values to a plurality of target patients. In such cases, after either assigning a value to the physiological feature of interest to the (current) target patient or after determining that the difference between the population measures is not statistically significant, the method 100 may continue in operation 180 to repeat operations 120 to 180 for a next target patient.

The method 100 comprises the use of two parameters - a threshold similarity (or distance) parameter and a statistical significance threshold parameter (e.g. threshold probability value or threshold significance value). These parameters may be predetermined, and may be selected by a user. That is, the accuracy of the imputed (e.g. assigned) values may be tailored by adjusting the values of these parameters. In some cases, a choice of distance measure may also be considered a parameter (e.g. a third parameter). For example, the choice of measuring a distance using a Gower distance, a Manhattan distance or a Jaccard distance may be considered as a distance measure parameter, which may be selected or adapted by a user.

The method 100 uses data of patients similar to the target patient in order to impute a missing value for the target patient. As such, the method implicitly corrects for the presence of different unknown groups of patients which may be associated with different mechanisms leading to the value to be imputed.

The method 100 provides a quantitative approach to assigning values to a physiological feature of interest of a target patient based on local data. The likelihood of the target patient having the specific value assigned may be supported by a p-value obtained from the statistical testing, providing a quantitative indication of confidence and probability to the imputation of the missing value, which can be output to and/or assessed by a user.

Additionally, the method 100 is explainable and transparent, as the additional features used to support the imputation (e.g. those of the identified subset of patients) are known and can be output. In some cases, the patients of the subset of recorded patients which are similar to the target patient may be output. That is, the patients whose recorded values for the physiological feature of interest were used to impute or assign a value for the target patient may be known and output. This information may be used to explain which patients and/or features are used to support the imputation. This information may then be used to identify any potential biases in the data.

Assigning a value to a physiological feature of interest for a target patient in the manner described herein may improve the completeness of the patient data. Complete patient data may be particularly suited for use with a variety of machine learning models, enabling a wider range of machine learning models to be used with greater accuracy. Moreover, missingness in data may limit the applicability of multivariate statistical analyses on a group of patients, since such approaches typically only use features which are available for all patients in the system. The method 100 may improve the robustness to unmeasured differences, for example by using a particular combination of additional features in its imputation, or the like.

The method 100 therefore does not require a set of training patients, in contrast to many mathematical or computational models which are commonly used for feature imputation. Since no training patients are required, there is a reduced risk of inaccurate modelling due to patients having the same medical condition but through different mechanisms. In other words, method 100 is model agnostic.

A specific example relating to the imputation of an ejection fraction for a target patient will now be described with reference to **Figure 2** and **Figures 3a** through **3c.** That is, the physiological feature of interest in this example is an ejection fraction. The missing value is therefore the value of the ejection fraction.

The example described with reference to Figure 2 relates to the identification or diagnosis of heart failure patients with preserved ejection fraction (HFpEF). Identifying features associated with HFpEF patients is important as it can uncover potential prognostic markers and be used to discover potential novel physio-pathological pathways which may be used to propose novel drug targets. HFpEF patients may fall into different subgroups (phenotypes), having potentially different driving factors. Diagnosis of sub-types based on ejection fraction can also be used for recommending of corresponding treatment options and/or prognosis. For these reasons, algorithms designed to give similar weight to features across a whole patient population have been of limited use. Research has shown that HFpEF patients may have different overlapping syndromes, making them inherently hard to identify.

Figure 2 illustrates a flowchart for this specific method, labelled 200. It is to be understood, however, that the method 200 is merely a more specific example of the implementation of method 100 described with reference to Figure 1. Many of the steps of method 200 are similar, if not identical, to the correspondingly numbered steps of method 100, and as such will not be described in detail. For example, method step 210 corresponds to the more general method step 110, and so on.

Method 200 comprises, in operation 210, accessing patient information for a plurality of recorded patients whose patient information includes a value of an ejection fraction. That is, the plurality of recorded patients may be patients who have been diagnosed with heart failure and whose ejection fraction is known and recorded. The patient information for the plurality of recorded patients further comprises values for one or more additional features, such as age, sex and blood pressure.

For example, patients from a large general database may be identified as heart failure (HF) patients based on one or more recorded diagnostic codes present in their patient data (e.g. ICD-10 codes I50 ("heart failure"), I11.0 ("hypertensive heart disease with congestive heart failure"), I13.0 ("hypertensive heart and renal disease with congestive heart failure") or I13.2 ("hypertensive heart and renal disease with both congestive heart failure and renal failure")), whose ejection fraction is known.

The recorded patients have a known ejection fraction value. The ejection fraction for each of the recorded patients is one of three values: reduced ejection fraction, mildly reduced ejection fraction, and preserved ejection fraction. If the ejection fraction is less than or equal to 30%, the value for the ejection fraction corresponds to heart failure with reduced ejection fraction (HFrEF). If the ejection fraction is greater than 30% but less than or equal to 50%, the value for the ejection fraction corresponds to heart failure with mildly reduced ejection fraction (HFmrEF). If the ejection fraction is greater than 50%, the value for the ejection fraction corresponds to heart failure with preserved ejection fraction (HFpEF). In some embodiments, the population may be split into only two classes, HFpEF and HFrEF with a boundary at 50% EF between the two classes or any other kind of decision rule, for example HFpEF for EF>50% and HFrEF for EF<40% or <30%..

Method 200 further comprises, in operation 220, obtaining target patient information for a target patient, for example a HF patient, whose ejection fraction is not known or recorded. That is, the target patient has a missing value for the ejection fraction. The target patient information comprises values for the one or more additional features, e.g. age, sex and blood pressure.

Method 200 further comprises, in operation 240, selecting a subset of the recorded patients which are similar to the target patient. In some cases, the subset may be selected by first determining a measure of similarity or a distance between each recorded patient of the plurality of recorded patients and the target patient based on the one or more additional features, as described with reference to operation 130. This may be done based on a distribution of recorded patients, e.g. distributed based on the one or more additional feature. A simplified case of such a distribution is illustrated in Figure 3a, purely for illustrative purposes.

Figure 3a shows a plurality of recorded patients, represented by the 16 circles 310 and 16 triangles 320. Recorded patients represented by the 16 circles have a reduced ejection fraction (HFrEF), whilst recorded patients represented by the 16 triangles have a preserved ejection fraction (HFpEF). In this simplified example, only two classes (HFrEF and HFpEF) are shown - although the principle applies for any number of classes.

The recorded patients are shown in Figure 3a in a distributed manner according to the values of the one or more additional features. In this simplified example, two additional features are used for this distribution - a first additional feature along the x-axis, and a second additional feature along the y-axis.

The target patient is also shown in this distribution, as illustrated by a square 330 in Figure 3b, based on the known values for the one or more additional features for the target patient.

The subset of recorded patients similar to the target patient may be determined by selecting all patients within a particular radius of the target patient, in the distribution. In the example of Figure 3b, this is illustrated by the hashed circle of radius d centered on the target patient 330. That is, in the example shown in Figure 3b, the subset of recorded patients similar to the target patient includes a total of 8 patients: 4 patients with HFrEF, represented by the circles, and 4 patients with HFpEF, represented by the triangles.

The value of the distance d, e.g. the radius used to select the subset of similar patients, may be predetermined or preselected. In some cases, the value of the distance d may be at least partially based on the type of distance used, for example a Jaccard distance, a Euclidean distance, or the like.

Continuing with the example illustrated in Figure 3b, the method 200 continues, in operation 242, by determining a frequency of occurrence of HFpEF in the subset of recorded patients similar to the target patient. As indicated above, the subset demarcated by the circle includes 4 patients whose ejection fraction value is HFpEF (e.g. preserved ejection fraction).

The method 200 continues, in operation 246, by determining a probability of obtaining at least the determined frequency of occurrence of HFpEF given a base probability. The base probability may correspond to a probability of HFpEF in the plurality of recorded patients. In this case, there are a total of 32 recorded patients (e.g. the total number of circles and triangles), of which 16 (e.g. the number of triangles) have a value of HFpEF.

The base probability, in this case, is therefore

$$p_{base} = \frac{16}{32} = 0.5$$

.

The probability of obtaining at least 4 patients with HFpEF out of the subset size of 8 patients given a base probability of 0.5 is therefore 0.64, using the one-tailed binomial test.

The method 200 then continues, in operation 250, to determine whether the probability of obtaining at least 4 patients with HFpEF out of the subset of 8 patients is statistically significant. In this example, the probability calculated in operation 246 ($p$ = 0.64) is compared to a threshold probability value of $p_{threshold}$ = 0.01.

Since the measured probability (p = 0.64) is not less than the threshold probability value of 0.01, the method 200 continues to operation 270, and assigns no value to the ejection fraction for the target patient. In other words, HFpEF is not an overrepresented category and an imputed value in this situation is not likely to be correct, so no value is assigned.

Referring now to the example illustrated in Figure 3c, in this case the target patient has a different position within the distribution, as illustrated by the square 330a.

In this case, the subset of patients within a radius of d from the target patient 330a comprises 7 triangles (patients with preserved ejection fraction, HFpEF) and no circles (patients with reduced ejection fraction, HFrEF).

The base probability is unchanged from the example of Figure 3b, as the total number and distribution of patients in the plurality of recorded patients is the same (32 total patients, 16 of which have reduced ejection fraction and 16 of which have preserved ejection fraction). The base probability is therefore still 0.5.

The probability of obtaining 7 patients with a preserved ejection fraction out of the subset of 7 patients given the base probability of 0.5 is 0.008, using the one-tailed binomial test. It is noted that, in this example, only two classes were used - HFrEF and HFpEF. However, if all three classes related to ejection fraction were used (e.g. HFrEF, HFmrEF and HFpEF), then a multivariate multinomial analysis, or a one-versus-rest binomial test may be used, for example.

When this is compared to the threshold probability value in operation 250, the probability of 0.008 is less than the threshold probability value of 0.01, that is, HFpEF is overrepresented in the subset of similar patients. The method 200 therefore continues to operation 260, in which the target patient is assigned a value of "preserved ejection fraction" for the ejection fraction feature.

In some cases, the calculated probability (or a statistical significance) may be output or included in the target patient information.

Operations 242, 246 and 250 may be collectively described as determining whether a particular value is overrepresented in the subgroup of recorded patients similar to the target patient. Any method of determining whether a value is overrepresented in a sample set may be used for this purpose.

In some cases, the method 200 optionally continues to operation 265, in which the target patient is diagnosed with having heart failure with preserved ejection fraction. The diagnosis may be output to a user, e.g. a healthcare provider such as a physician, via any user-perceptible means, e.g. visually and/or audibly. A level of confidence, e.g. based on the determined probability (in this case having a value of 0.008) may be output with the diagnosis. The diagnosis may be used to recommend one or more treatment options, and/or to provide a prognosis, either automatically or by a healthcare professional. It will be understood that diagnosis and recommendation of treatment options may proceed in analogous manner for a target patient for which HFrEF is overrepresented in the subset of patients.

The described methods use two parameters that can be tuned, the distance threshold for inclusion of patients in the subset and the statistical significance threshold for the assignment of a category / diagnosis. Another "parameter" is the selection of the distance metric. These parameters can be selected and/or tuned based on experience or in any other way known to a person skilled in the field of data science or machine learning. For example, in some implementations, the parameters can be tuned / selected using a validation set of target patients for which the EF or EF category / diagnosis is known. In some implementations, performance of the method can be evaluated using a separate test set of target patients for which the EF or EF category / diagnosis is known.

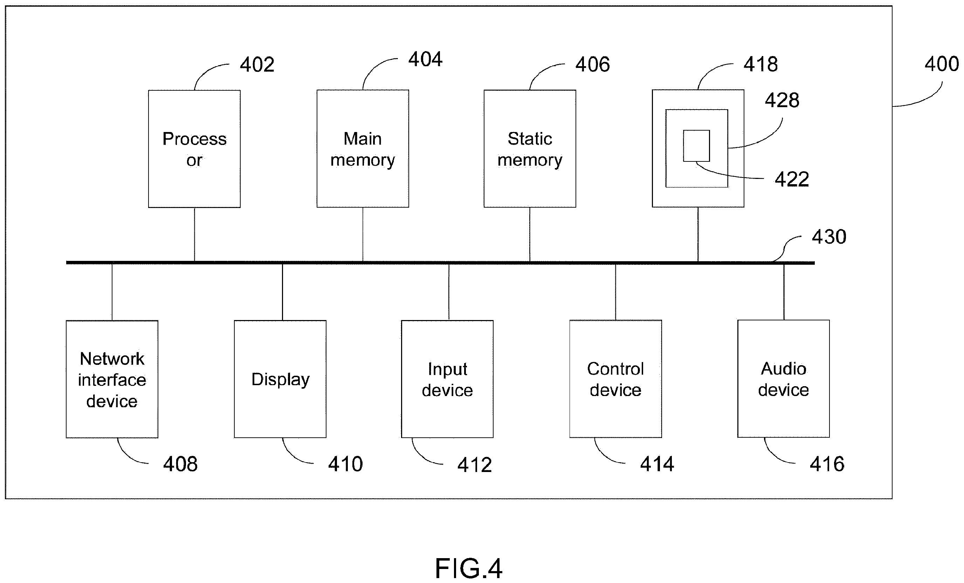

Figure 4 illustrates a block diagram of one implementation of a computing device 400 within which a set of instructions, for causing the computing device to perform any one or more of the methodologies discussed herein, may be executed. In alternative implementations, the computing device may be connected (e.g., networked) to other machines in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 400 includes a processing device 402, a main memory 404 (e.g., read-only memory (ROM), flash memory, dynamic random access memory (DRAM) such as synchronous DRAM (SDRAM) or Rambus DRAM (RDRAM), etc.), a static memory 406 (e.g., flash memory, static random access memory (SRAM), etc.), and a secondary memory (e.g., a data storage device 418), which communicate with each other via a bus 430.

Processing device 402 represents one or more general-purpose processors such as a microprocessor, central processing unit, or the like. More particularly, the processing device 402 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 402 may also be one or more special-purpose processing devices such as an application specific integrated circuit (ASIC), a field programmable gate array (FPGA), a digital signal processor (DSP), network processor, or the like. Processing device 402 is configured to execute the processing logic (instructions 422) for performing the operations and steps discussed herein.

The computing device 400 may further include a network interface device 408. The computing device 400 also may include a video display unit 410 (e.g., a liquid crystal display (LCD) or a cathode ray tube (CRT)), an alphanumeric input device 412 (e.g., a keyboard or touchscreen), a cursor control device 414 (e.g., a mouse or touchscreen), and an audio device 416 (e.g., a speaker).

The data storage device 418 may include one or more machine-readable storage media (or more specifically one or more non-transitory computer-readable storage media) 428 on which is stored one or more sets of instructions 422 embodying any one or more of the methodologies or functions described herein. The instructions 422 may also reside, completely or at least partially, within the main memory 404 and/or within the processing device 402 during execution thereof by the computer system 400, the main memory 404 and the processing device 402 also constituting computer-readable storage media.

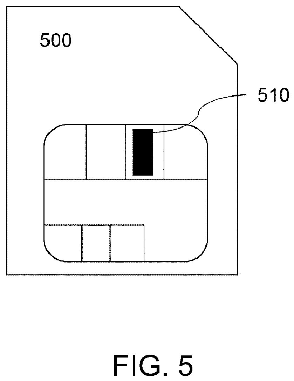

The various methods described above may be implemented by a computer program. The computer program may include computer code arranged to instruct a computer to perform the functions of one or more of the various methods described above. The computer program and/or the code 510 for performing such methods may be provided to an apparatus, such as a computer, on one or more computer readable media or, more generally, a computer program product, depicted in **Figure** 5. The computer readable media 500 may be transitory or non-transitory. The one or more computer readable media 500 could be, for example, an electronic, magnetic, optical, electromagnetic, infrared, or semiconductor system, or a propagation medium for data transmission, for example for downloading the code over the Internet. Alternatively, the one or more computer readable media 500 could take the form of one or more physical computer readable media such as semiconductor or solid state memory, magnetic tape, a removable computer diskette, a flash drive, a random access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "comparing ", "assigning", "finding," "imputing", "identifying" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

**Claims**

1. A computer-implemented method for determining a missing value of a physiological feature of interest for a target patient, the method comprising:

   accessing patient information for a plurality of recorded patients, the patient information comprising, for each patient of the plurality of recorded patients, recorded values for a plurality of features, the plurality of features including the physiological feature of interest and one or more additional features;
   obtaining target patient information for the target patient, the target patient information comprising values for of the one or more additional features;
   for each patient of the plurality of recorded patients, determining a respective measure of similarity between said patient and the target patient based on the one or more additional features;
   selecting a subset of patients of the plurality of recorded patients that are similar to the target patient based on the respective one or more measures of similarity;
   determining if a difference between a population measure of the recorded values corresponding to the physiological feature of interest for the subset of the plurality of recorded patients that are similar to the target patient and the population measure of the recorded values corresponding to the physiological feature of interest for the plurality of recorded patients is statistically significant using a statistical test; and

in response to determining that the difference is statistically significant, assigning a value to the physiological feature of interest for the target patient based on the recorded values of the physiological feature of interest for the subset of patients of the plurality of recorded patients that are similar to the target patient.

2. The method of claim 1, wherein the physiological feature of interest is a categorical feature.

3. The method of claim 2, wherein assigning the value to the physiological feature of interest for the target patient comprises assigning the category of the categorical feature which is overrepresented in the subset of patients of the plurality of recorded patients that are similar to the target patient as the missing value.

4. The method of any preceding claim, wherein the missing value indicates whether a numeric value of a physiological measurement corresponding to the physiological feature of interest is within a predefined numerical range.

5. The method of any preceding claim, wherein the physiological feature of interest is an ejection fraction for heart failure, the ejection fraction being a volumetric fraction of fluid ejected from a chamber of a heart with each contraction.

6. The method of claim 5, wherein the missing value indicates if the ejection fraction is a preserved ejection fraction, a reduced ejection fraction or a mildly reduced ejection fraction.

7. The method of claim 5, wherein the missing value indicates if the ejection fraction is a preserved ejection fraction or a reduced ejection fraction.

8. The method of claim 5 or 7, wherein the target patient is a heart-failure patient, for example a patient with chronic heart failure, and the method comprises diagnosing the target patient as one of preserved ejection fraction and reduced ejection fraction heart failure patient.

9. The method of claim 5 or 6, wherein the target patient is a heart-failure patient, for example a patient with chronic heart failure, and the method comprises diagnosing the target patient as one of preserved ejection fraction, mildly reduced ejection fraction and reduced ejection fraction heart failure patient.

10. The method of claim 8 or 9, comprising recommending a treatment or making a prognosis based on the diagnosis.

11. The method of any preceding claim, wherein the one or more measures of similarity comprise a distance between the respective one or more additional features in a corresponding feature space.

12. The method of claim 11, wherein the distance is determined using at least one of a Jaccard distance, a Manhattan distance and a Gower distance.

13. A transitory or non-transitory computer-readable medium comprising instructions which, when executed by a computer, cause the computer to carry out the method of any one of claims 1 to 12.

14. A device comprising:

a transmission subsystem configured to transmit data;
a receiving subsystem configured to receive data;
one or more processors;
a memory comprising instructions which, when executed, cause the one or more processors to execute the method of any one of claims 1 to 12.

FIG. 1

200

FIG. 2

FIG. 3a

FIG. 3b

FIG. 3c

FIG.4

FIG. 5

Europäisches
Patentamt
European
Patent Office
Office européen
des brevets

# EUROPEAN SEARCH REPORT

Application Number

EP 21 21 7609

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/220835 A1 (CHUNG WAYNE [US]) 30 August 2012 (2012-08-30) * paragraphs [0033], [0025], [0046] * | 1-14 | INV. G16H10/60 |
| A | JP 2021 186686 A (BIOSENSE WEBSTER ISRAEL LTD) 13 December 2021 (2021-12-13) * the whole document * | 1-14 | |
| A | FRANCESCO VIOLI ET AL: "Vitamin E for the Treatment of Cardiovascular Disease: Is There a Future?", ANNALS OF THE NEW YORK ACADEMY OF SCIENCES, NEW YORK ACADEMY OF SCIENCES, US, vol. 1031, no. 1, 12 January 2006 (2006-01-12), pages 292-304, XP071401476, ISSN: 0077-8923, DOI: 10.1196/ANNALS.1331.029 * the whole document * | 1-14 | |

TECHNICAL FIELDS
SEARCHED      (IPC)

G16H

The present search report has been drawn up for all claims

| Place of search | Date of completion of the search | Examiner |
|---|---|---|
| Munich | 3 June 2022 | Laub, Christoph |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 21 7609

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2022

| Patent document cited in search report | | Publication date | Patent family member(s) | | Publication date |
|---|---|---|---|---|---|
| US 2012220835 | A1 | 30-08-2012 | US 2012220835 | A1 | 30-08-2012 |
| | | | WO 2012112407 | A1 | 23-08-2012 |
| JP 2021186686 | A | 13-12-2021 | CN 113749670 | A | 07-12-2021 |
| | | | EP 3928702 | A2 | 29-12-2021 |
| | | | JP 2021186686 | A | 13-12-2021 |
| | | | US 2021378579 | A1 | 09-12-2021 |

EPO FORM P0459

For more details about this annex : see Official Journal of the European Patent Office, No. 12/82